# EUROPEAN PATENT APPLICATION

(11) **EP 2 594 628 A1**
(43) Date of publication of application: **22.05.2013**
(21) Application number: 11806698.4
(22) Date of filing: 07.07.2011
(51) Int. Cl.: C12M 1/00

(54) **CELL CULTURE VESSEL AND CELL CULTURE DEVICE**

(30) Priority: 25.04.2011 JP 2011097149; 16.07.2010 JP 2010161863
(71) Applicant: Hitachi, Ltd., Chiyoda-ku Tokyo 100-8280 (JP)
(72) Inventor: NAKAJIMA Ryota, Chiyoda-ku, Tokyo 100-8220 (JP); MORIYA Noboru, Chiyoda-ku, Tokyo 100-8220 (JP); KOBAYASHI Toyoshige, Chiyoda-ku, Tokyo 100-8220 (JP); MATSUOKA Shizu, Chiyoda-ku, Tokyo 100-8220 (JP); NOZAKI Takayuki, Chiyoda-ku, Tokyo 100-8220 (JP); MORI Keisuke, Chiyoda-ku, Tokyo 100-8220 (JP)
(74) Representative: Strehl Schübel-Hopf & Partner
(86) International application number: PCT/JP2011/065626
(87) International publication number: WO 2012/008368

(57) **Abstract**

Disclosed is a cell culture vessel that can prevent liquid leakage from the cell culture space and entry of particles containing microorganisms from outside the cell culture space, and that can prepare regenerated tissue in a manner that is safe and results in peace of mind. The cell culture vessel is mounted to a cell culture device, holds/cultures cells, and is characterized by being provided with: a culture solution holding section that holds a culture solution, a protruding structure section that is for supplying and discharging the aforementioned culture solution; and a culture solution duct that passes from the aforementioned protruding structure section to the aforementioned culture solution holding section.

## Description

### Technical Field

The present invention relates to a cell culture vessel and a cell culture device using the same.

### Background Art

In regenerative medicine that treats a disease by using one's own cells or another individual's cells, cells collected from a living body are often cultured to increase the number of cells or form tissue in an appropriate form, and then the cells or tissue are used for treatment. Cells used for treatment needs to be cultured in accordance with GMP (Good Manufacturing Practice) in a clean room for culturing cells, which is called a Cell Processing Center (CPC). The problem herein lies in the fact that cell culture is performed by work of an engineer, and thus much effort and cost are needed to prepare cells for a patient, and in the fact that there is a risk of being biologically contaminated due to manual operation.

As a means for addressing these problems, devices for automating the cell culture process in a closed system have been developed. This means that the automation of the cell culture process and the reduction in biological contamination risk are achieved by using a closed-system culture vessel which does not need any operation that opens and closes the cover of the culture vessel.

On the other hand, in cells, there are cell strains that need vegetative cells called feeder cells in the process of proliferating cells and cell strains that do not need the vegetative cells. Cell strains, such as ES (Embryonic Stem) cells or iPS (Induced Pluripotent Stem) cells, which are highlighted in the regenerative medicine, skin epithelial cells, corneal epithelial cells and oral mucosal epithelial cells, often need feeder cells. When cultured cells are used for treatment, it is preferred that feeder cells and cells used for treatment are cultured while being separated from each other. That is, it is preferred that cells are cultured in a cell culture vessel having a double-layered culture layer. However, closed-system cell culture devices which have been developed until now have a device configuration corresponding to a cell culture vessel having a one-layered culture layer, and thus it was difficult to achieve a double-layered culture.

As a means for addressing the problem, culture devices as shown in Patent Literature 1 and 2 have been suggested. In the device configuration, it is possible to automatically culture cell strains such as ES cells or iPS cells, skin epithelial cells, corneal epithelial cells and oral mucosal epithelial cells in a closed-system by using a cell culture vessel having a double-layered culture layer, for example, as shown in Patent Literature 3 and 4.

### Citation List

### Patent Literature

Patent Literature 1: Japanese Patent Application Laid-Open Publication No. 2006-149237
Patent Literature 2: Japanese Patent Application Laid-Open Publication No. 2008-271915
Patent Literature 3: Japanese Patent Application Laid-Open Publication No. 2008-271912
Patent Literature 4: Japanese Patent Application Laid-Open Publication No. 2008-271911

### Summary of Invention

### Technical Problem

However, when an automated culture is performed by using a cell culture vessel having a double-layered culture layer as shown in Patent Literature 3 and 4, there are problems described below.

When a waste liquid is automatically transferred by using a closed-system culture vessel described in Patent Literature 3, for example, Patent Literature 1 and 2 describe methods of connecting a means for supplying a culture solution provided in a device joint to an elastic member provided in the cell culture vessel to transfer the waste culture solution (see claim 1 of Patent Literature 1 and claim 1 of Patent Literature 2).

In these methods, it was a problem that liquid leakage occurs from a vent hole produced by passing a pipe for transferring a waste solution through a slit of the elastic member, and particles containing microorganisms from outside the cell culture space through the vent hole are incorporated.

Further, after cells are cultured in a closed-system culture vessel described in Patent Literature 3, it is essentially required that the cell culture vessel is ejected from a cell culture device, a culture solution in the cell culture vessel is discharged in a safety cabinet immediately before cells are used, and cells are recovered. In releasing the culture solution from the cell culture vessel, it is necessary to insert a certain pipe into an elastic member provided in the cell culture vessel manually. It was a problem in that the danger of biological contamination is increased by means of manual manipulation in this manner.

The present invention has been made in consideration of the problem, and it is an object to provide a cell culture vessel and a cell culture device using the same so as to address these problems.

### Solution to Problem

The cell culture vessel of the present invention to achieve the object is mounted to a cell culture device, and provides a cell culture vessel with a configuration including a culture solution holding section that holds a culture solution, a protruding structure section that is for supplying and discharging the culture solution; and a culture solution duct that passes from the protruding structure section to the culture solution holding section in the cell culture vessel of holding and culturing cells.

Further, provided is a cell culture device of culturing cells in the cell culture vessel, the cell culture device including: a culture stage that holds the cell culture vessel, a culture solution storage section that stores a cell solution, a waste solution receiving section that collects a waste solution, a liquid transferring pipe that supplies the culture solution from the culture solution storage section to the cell culture vessel, and a waste solution pipe that recovers the waste solution form the cell culture vessel to transfer the waste solution to the waster solution receiving section, in which the cell culture vessel includes a culture solution holding section that holds the culture solution and a protruding structure section that supplies and discharges the culture solution and a culture solution duct that passes from the protruding structure section to the culture holding section, and the protruding structure section is connected to one end of each of the liquid transferring pipe and the waste solution pipe.

### Advantageous Effects of Invention

When the cell culture vessel according to the present invention is used, it is possible to automatically culture cells in a completely closed-system. By culturing cells in a completely closed-system, entry of particles containing microorganisms from outside the cell culture space may be suppressed and cells may be cultured in a manner that is safe and results in peace of mind.

Further, even when the culture solution is discharged from the cell culture vessel after the completion of cell culture, entry of particles containing microorganisms from outside the cell culture space may be suppressed and cells after the culture may be recovered in a manner that is safe.

### Brief Description of Drawings

[FIGS. 1] FIGS. 1 are views illustrating a cell culture vessel of Example 1.
[FIG. 2] FIG. 2 is a view illustrating how a tube is connected to the cell culture vessel according to Example 1.
[FIG. 3] FIG. 3 is a view illustrating various protruding structures of the cell culture vessel according to Example 1.
[FIG. 4] FIG. 4 is a view illustrating a cell culture device having the cell culture vessel according to Example 1.
[FIG. 5] FIG. 5 is a view illustrating a method of releasing the connection of the cell culture device and the cell culture vessel after the completion of the cell culture according to Example 1.
[FIG. 6] FIG. 6 is a view illustrating a method of discharging a culture solution from the cell culture vessel according to Example 1.
[FIG. 7] FIG. 7 is a view illustrating an order of recovering a regenerated tissue from the cell culture vessel according to Example 1.
[FIG. 8] FIG. 8 is a view illustrating how a gas permeable film is welded to the cell culture vessel according to Example 1.
[FIGS. 9] FIGS. 9 are views illustrating an example of a one-layered cell culture vessel according to a modified example of Example 1.
[FIGS. 10] FIGS. 10 are views illustrating another example of a one-layered cell culture vessel according to a modified example of Example 1.
[FIGS. 11] FIGS. 11 are views illustrating another example of the position of a protruding structure in a cell culture vessel according to a modified example of Example 1.
[FIG. 12] FIG. 12 is a view illustrating an appearance of a cell culture vessel frame body manufactured in Specific Example 1 of Example 1.
[FIG. 13] FIG. 13 is a view illustrating an appearance of the state in which a gas permeable film and the like are welded to the cell culture vessel frame body manufactured in Specific Example 1 of Example 1 to be sealed while holding a culture solution.
[FIGS. 14] FIGS. 14 are views illustrating phase contrast microscope images and appearances of peeled and recovered cell sheets in Specific Example 1 and Comparative Example 1 of Example 1.
[FIG. 15] FIG. 15 is a view illustrating the number of cells included in a corneal epithelial tissue in Specific Example 1 and Comparative Example 1 of Example 1.
[FIG. 16] FIG. 16 is a view illustrating a hematoxylin-eosin (HE) staining image of a corneal epithelial tissue fragment in Specific Example 1 and Comparative Example 1 of Example 1.
[FIGS. 17] FIGS. 17 are views illustrating an immunohistochemical staining image of a corneal epithelial tissue fragment in Specific Example 1 and Comparative Example 1 of Example 1.
[FIG. 18] FIG. 18 is a view illustrating appearances of peeled and recovered cell sheets in Specific Example 2 and Comparative Example 2 of Example 1.
[FIGS. 19] FIGS. 19 are views illustrating a hematoxylin-eosin (HE) staining image and an immunohistochemical staining image of a corneal epithelial tissue fragment in Specific Example 2 and Comparative Example 2 of Example 1.
[FIGS. 20] FIGS. 20 are views illustrating an immunohistochemical staining image of a corneal epithelial tissue fragment and a p63 positive cell rate in Specific Example 2 and Comparative Example 2 of Example 1.
[FIGS. 21] FIGS. 21 are views illustrating a colony detection image and a colony formation rate in Specific Example 2 and Comparative Example 2 of Example 1.
[FIG. 22] FIG. 22 is a view illustrating a cross section of a cell culture vessel according to Example 2.
[FIGS. 23] FIGS. 23 are views illustrating FIG. 23 (a) a three-dimensional shape and FIG. 23 (b) cross section of the cell culture vessel of Example 2.
[FIGS. 24] FIGS. 24 are views illustrating photos of FIG. 24 (a) an upper portion and FIG. 24 (b) an oblique side of the cell culture vessel of Example 2.
[FIG. 25] FIG. 25 is a view illustrating a cross section of a cell culture vessel according to Example 3.

### Description of Embodiments

Hereinafter, various Examples of the present invention will be described with reference to the accompanying drawings. However, these Examples are only an example for implementing the present invention, and do not limit the technical scope of the present invention. Further, the same reference numerals are given to components common in each drawing. In addition, in the present specification, a culture solution is called a culture medium in some cases.

### Example 1

### <Structure of Cell Culture Vessel>

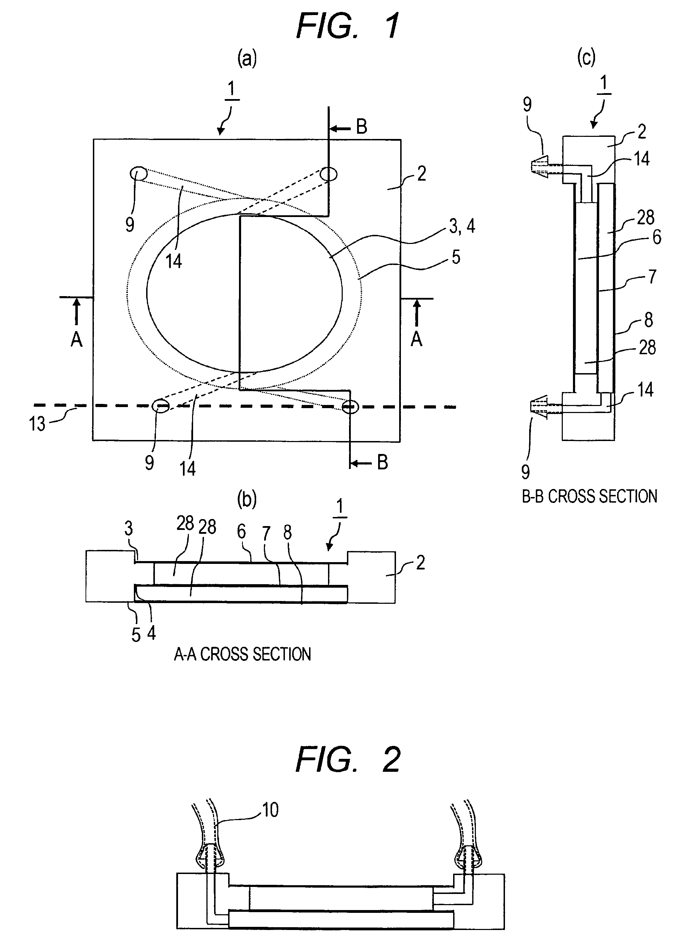

FIGS. 1 are views illustrating an example of the structure of a cell culture vessel in Example 1.

FIG. 1 (a) is a top view, FIG. 1 (b) is an A-A cross-sectional view thereof, and FIG. 1 (c) is a B-B cross-sectional view thereof. A cell culture vessel 1 is a regular quadrilateral and flat-shaped vessel and formed of a plastic having plasticity and rigidity, such as polycarbonate, polystyrene, polypropylene and the like. A frame body 2 is formed by injection molding, and circular sections 3, 4 and 5 are formed inside thereof. Moreover, a gas permeable film 6, a material permeable film 7, and a gas permeable film 8 are welded to the circular sections 3, 4 and 5, respectively. The welding of the gas permeable films 6 and 8 and the material permeable film 7 to the circular sections 3, 5 and 4 is preferably a thermal welding or ultrasonic welding, which does not use an adhesive that affects the viability of cells. In the present example, a double-layered culture solution holding section 28 having an approximately circular shape is configured by each of the layers and the frame body 2.

In the same drawing, reference numeral 9 is a pair of protruding structure sections that supplies and discharges a culture solution to the culture solution holding section 28. The protruding structure section 9 protrudes in a direction vertical to the film surfaces of the gas permeable films 6 and 8 from the upper surface of the frame body 2 of the cell culture vessel 1. Reference numeral 14 is a pair of culture solution ducts from the protruding structure section 9 to the culture solution holding section 28 which is a circular culture space. The pair of culture solution ducts 14 are connected to an approximately circular shaped profile positions facing each other in each layer of the culture solution holding section 28. As described below, when the cell culture vessel 1 is made vertical, this configuration is for allowing the pair of culture solution ducts 14 to be connected to the uppermost section and bottom most section of the culture space. Further, the culture solution holding section 28 may also be formed in an approximately elliptical shape by making the circular sections 3, 4 and 5 into an elliptical portion.

When the cell culture vessel 1 is used for the purpose of culturing cell strains that require nutrient from the lower layer of the cell culture vessel 1 in the upper layer thereof, the average pore diameter of the material permeable film 7 may be a size that passes through proteins and the like, but does not pass through cells.

Materials for the gas permeable films 6 and 8 and the material permeable film 7 may be materials which have a gas permeable property and are transparent so as to make the observation of the cell culture possible, such as polycarbonate, polystyrene and the like, and are not limited thereto. When objects are welded to each other by means of thermal welding and ultrasonic welding, materials for the frame body 2, the gas permeable films 6 and 8, and the material permeable film 7 may be a material having the same or a similar melting point.

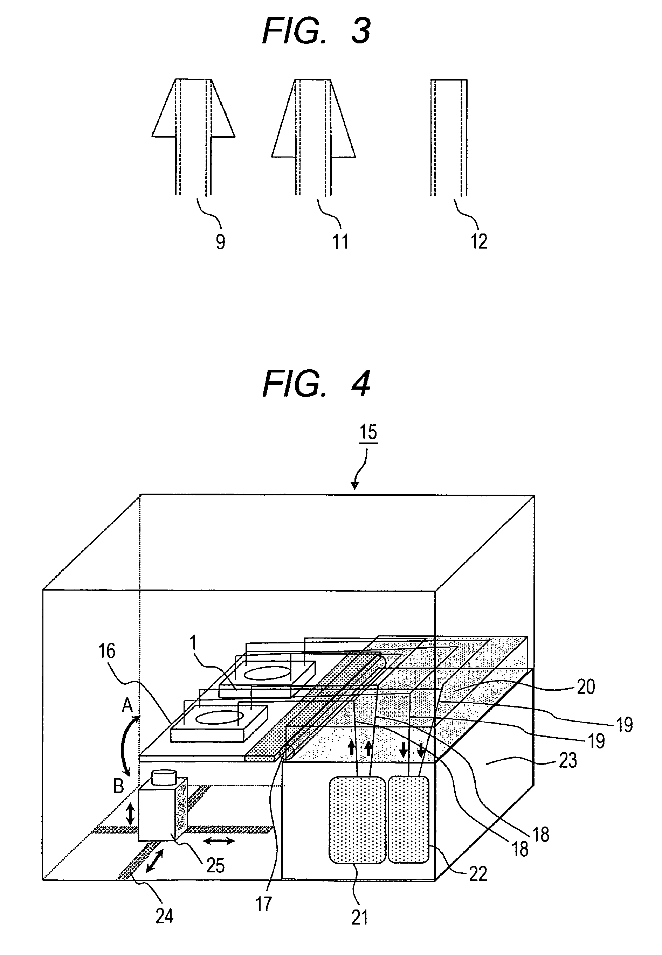

As shown in FIG. 2, the pair of protruding structure sections 9 provided in the cell culture vessel 1 are integrally molded into the cell culture vessel 1, and may be connected to a tube 10 formed of an elastic member. When the internal diameter of the tube 10 is the same as that of the protruding structure section 9, liquid leakage does not occur even when a solution is passed through while connecting the tube 10 to the protruding structure section 9 as in FIG. 2. The protruding structure section 9 has a trapezoidal tip, but is not limited to the shape, and may be a shape as long as the shape may allow the structure section 9 to be connected to the tube 10 such as the protruding structures 11 and 12 in FIG. 3. The height of the protruding structure section 9 is not particularly limited, but may be a height necessary to connect the protruding structure section 9 to the tube 10 from the viewpoint of usability.

The cell culture vessel 1 having such a structure in the present Example makes it possible to culture cells in a closed space by filling the culture solution holding section 28 formed by the material permeable film 7 and the gas permeable films 6 and 8 provided in the frame body 2 with a culture solution. When the culture solution holding section 28 is filled with a culture solution, in order not to generate bubbles, the culture vessel 1 is erected such that one protruding structure section 9 forming a pair thereof is located at the bottom thereof, the culture solution is injected from the protruding structure section 9 on the bottom side, and the culture solution is discharged from the protruding structure section 9 on the upper side. Therefore, in the case of the cell culture vessel 1 having a double-layered structure, in order to efficiently inject a solution into the upper and lower layers, it is necessary to maintain the protruding structure section 9 of the cell culture vessel as shown in FIGS. 1, that is, two protruding structures on the upper layer and two protruding structures on the lower layer, horizontally. Further, the culture solution duct 14 from the protruding structure section 9 to the culture solution holding section 28 which is a circular culture space is connected to the uppermost section and the bottom most section of the culture space when the cell culture vessel 1 is made vertical. Accordingly, the culture solution may be efficiently injected and discharged. In addition, as described above, the culture solution holding section 28 in the middle of the cell culture vessel 1 according to the present example is composed of a space having a circular shape or elliptical shape, such as an approximately cylinder, elliptical cylinder or the like. This is for recovering a circular or elliptical culture object, such as, for example, the cornea and the like in a state of a circular or elliptical shape.

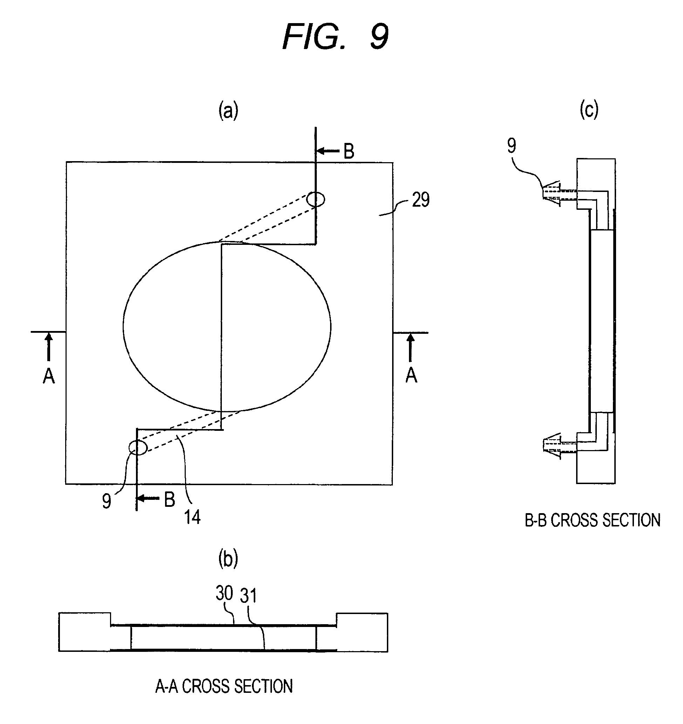

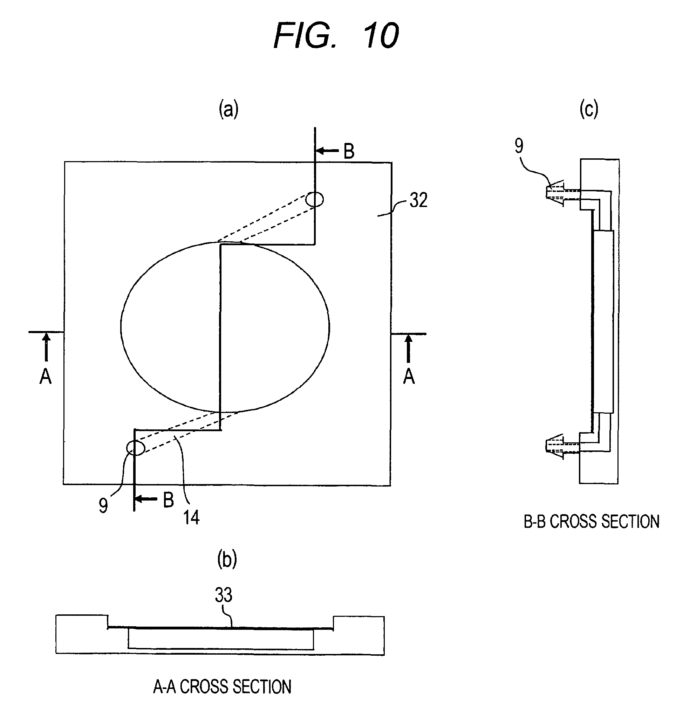

Further, the cell culture vessel 1 in FIGS. 1 does not need to be a double-layer shape, and various modified examples are constituted. For example, according to the cell strain to be cultured, the cell culture vessel 1 may also be a one-layered cell culture vessel in which gas permeable films 30 and 31 are welded to the frame body 29 as shown in FIGS. 9. Further, the one-layered cell culture vessel may also be a structure in which one gas permeable film 33 is only welded by molding as in the frame body 32 having a bottom section in FIGS. 10.

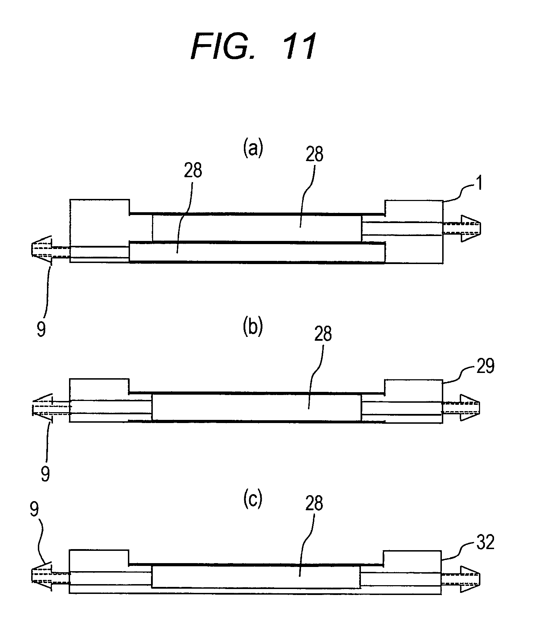

In addition, the protruding structure sections 9 of the cell culture vessels 1 in various modified examples shown in FIGS. 1, 9 and 10 need not be installed on the upper surface of the frame body 2 which is located over each corresponding culture solution holding section 28, and may also be located on the side surfaces of the frame bodies 2 of the cell culture vessels 1, 29 and 32 as in FIGS. 11.

Subsequently, an example of the configuration of the cell culture device using various cell culture vessels which have been described will be described.

### <Configuration of Cell Culture Device>

FIG. 4 is a view schematically illustrating the entire configuration of a cell culture device 15 in which the cell culture vessel 1 including a protruding structure capable of being connected to a tube which is a liquid transferring pipe or a waste solution pipe is disposed in the inside thereof as a cell culture device to which the cell culture vessel of the present example is applied.

In FIG. 4, the cell culture device 15 has a culture stage 16 which holds the cell culture vessel 1 and an actuating section 17 for rotating the culture stage 16. Further, the liquid transferring pipe 18 and the waste solution pipe 19, which are linked to the cell culture vessel, are connected to a culture solution storage section 21 and a waste solution receiving section 22 through a control section 20. The culture solution storage section 21 and the waste solution receiving section 22 are housed in a cooling box 23 set at, for example, 4°C. In addition, the culture solution is suitably warmed to 37°C by means of a heater not shown and the like in the control section 20, and then supplied to the cell culture vessel 1.

A cell observing apparatus 25 including a ZYX movable axis 24 is provided on or below the culture stage 16, and may monitor and record the state of cells which are cultured if necessary.

Further, filling the cell culture vessel 1 with the culture solution is performed in the following manner, such that bubbles are not generated in the cell solution vessel. That is, the culture stage 16 is erected vertically in the arrow A direction by means of the actuating section 17, such that the liquid transferring pipe 18 linked to the cell culture vessel 1 is located at the bottom thereof, and the cell culture vessel 1 is filled with a mixed solution of cells and the culture solution. The cell culture vessel is filled with cells and the culture solution, and then cells are cultured at predetermined temperature, humidity, gas composition and concentration for a predetermined time while the culture stage 16 is maintained by means of the actuating section 17 horizontally. During the culture, when the culture medium is exchanged with a fresh culture medium, the culture stage 16 is erected vertically in the arrow B direction by means of the actuating section 17, such that the waste solution pipe 19 linked to the cell culture vessel 1 is located at the bottom thereof, and the culture medium is sucked from the cell culture vessel 1. After the suction, the culture stage 16 is erected vertically, such that the liquid transferring pipe 18 linked to the cell culture vessel 1 is located at the bottom thereof, and the cell culture vessel 1 is filled with the culture medium. The cell culture vessel is filled with the culture medium, and then cells are cultured again at a predetermined temperature, humidity, gas composition and concentration for a predetermined time while the culture stage 16 is again maintained in parallel by the actuating section 17.

### <Release Connection of Cell Culture Device and Cell Culture Vessel after Completion of Cell Culture>

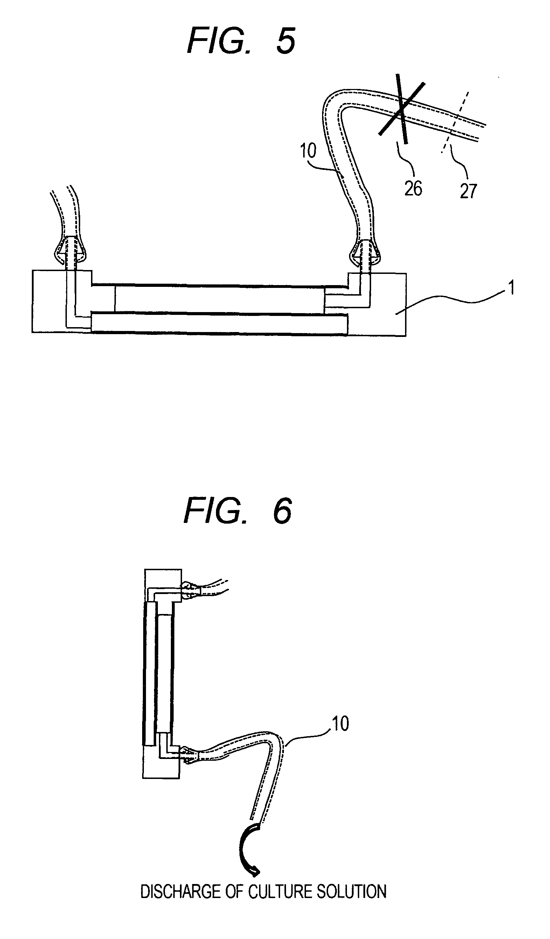

FIG. 5 is a view illustrating a method of releasing the connection of the cell culture device 15 and the cell culture vessel 1 after the completion of the cell culture in the present example. The cell culture vessel 1 may be taken out from the cell culture device 15 by blocking the middle of the tube 10 which is a liquid transferring pipe or waste solution pipe by means of a member 26 which closes the tube 10, such as a clamp or the like which is connected to the protruding structure section 9 of the cell culture vessel 1 and cutting a blocked tip 27 by means of a pair of sterilized scissors and the like.

The method of taking out the vessel is not limited to the method described above, and may be a method of providing a member capable of implementing a closed system in the middle of the tube and releasing the connection of the cell culture vessel and the cell culture device.

### <Discharge of Culture Solution from Cell Culture Vessel>

FIG. 6 is a view illustrating a method of discharging a culture solution from a cell culture vessel 1 which connection is released from the cell culture device 15. The culture solution may be simply discharged by inclining the cell culture vessel such that the protruding structure on the side in which the culture solution is to be discharged is located at the bottom thereof. The culture solution may be discharged immediately before a regenerated tissue manufactured is needed in order to prevent the regenerated tissue from being dried.

### <Recovery of Regenerated Tissue from Cell Culture Vessel>

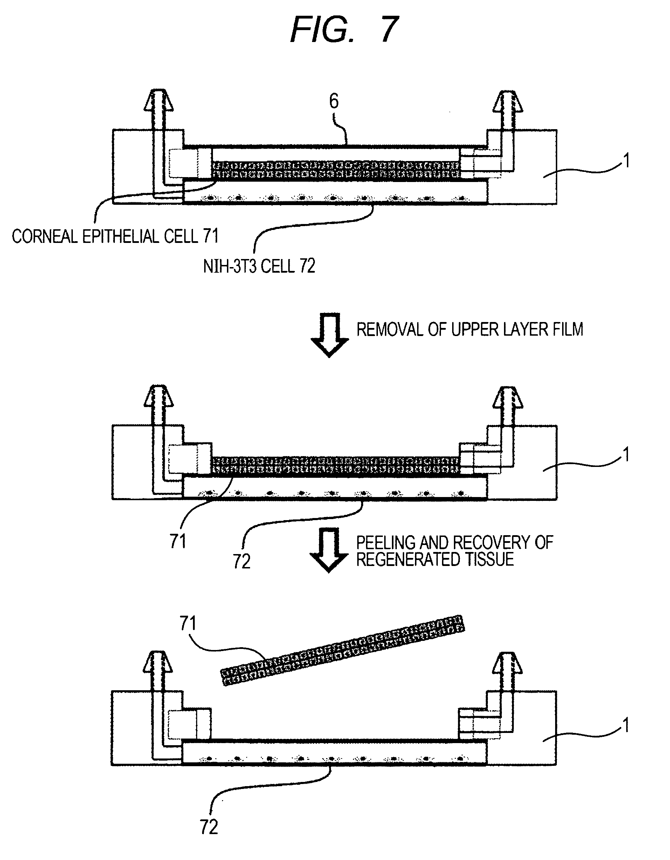

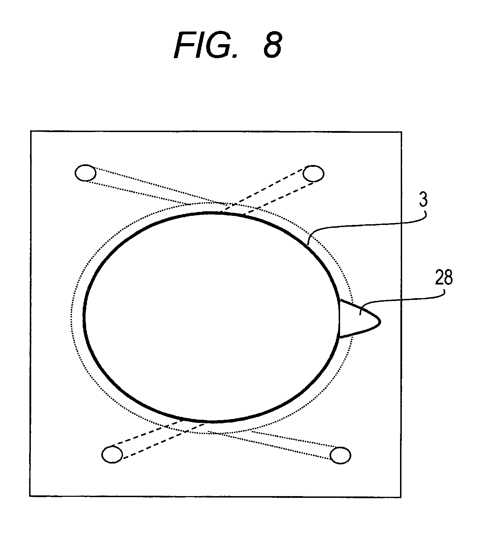

FIG. 7 is a view illustrating an order of recovering a regenerated tissue from the cell culture vessel. In the same drawing, Reference numerals 71 and 72 represent a corneal epithelial cell and an NIH-3T3 cell, respectively. First, a connected tube from the cell culture vessel from which the culture solution is discharged is taken to remove a gas permeable film 6 welded to the upper layer of the cell culture vessel 1 by means of a cutter knife and the like. Thereafter, the corneal epithelial cell 71 which is a regenerated tissue is washed with a saline solution and the like, and then the regenerated tissue may be recovered. As in the modified example shown in FIG. 8, when the gas permeable film 28 having a protruding section in which a part of the shape thereof protrudes into the circular section 3 of the cell culture vessel is welded, it is possible to remove the gas permeable film 28 by lifting the gas permeable film 28 by means of a pair of tweezers without cutting out the gas permeable film. Further, the protruding shape may be a shape which may be lifted by means of a pair of tweezers, and is not limited to the shape in FIG. 8.

As a method of recovering a corneal epithelial cell 71 which is a regenerated tissue, a method of using dispase may be used or the corneal epithelial cell 71 may be recovered from the cell culture vessel 1 while maintaining the tissue shape by including fibrin gel, amnion, a temperature-responsive polymer or the like on the surface of the cell culture in advance, but the recovery method is not limited to the methods.

Hereinafter, specific examples of a method of preparing a corneal epithelial tissue by the corneal epithelial cell culture using the cell culture vessel of the present example and the results thereof will be described.

### (Specific Example 1)

### <Method of Manufacturing Closed-System Cell Culture Vessel>

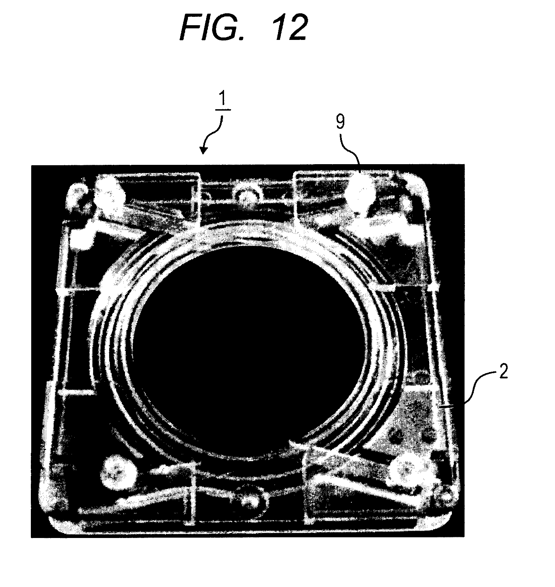

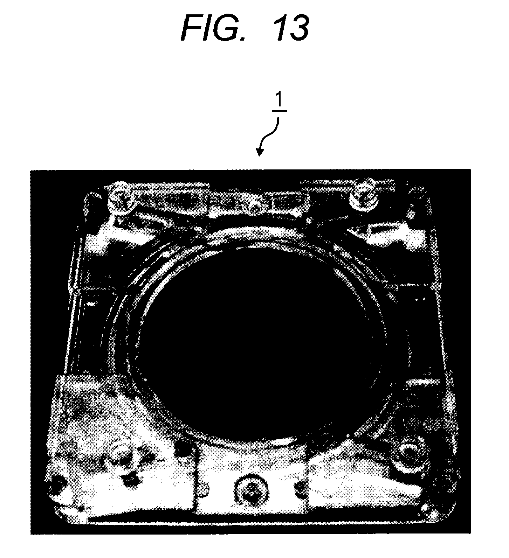

First, the frame body 2 as shown in FIGS. 1 was manufactured by injection molding (polycarbonate was used as a material). FIG. 12 illustrates an appearance of the frame body 2 actually manufactured. By means of an ultrasonic welding method, a gas permeable film was welded to the circular sections 3 and 5 in FIGS. 1 and a material permeable film was welded to the circular section 4 in FIGS. 1 (2000Xdt 40:0.8 manufactured by Branson Corp. was used as an ultrasonic welder). The film welded to the circular sections 4 and 5 in FIGS. 1 was subjected to hydrophilic treatment with a plasma device (PC-300 manufactured by Samco Corp. was used as the plasma device). FIG. 13 illustrates an appearance of the vessel 1 in which the culture solution is held after each film is welded.

### <Method of Culturing Corneal Epithelial Cells>

Subsequently, the method of culturing corneal epithelial cells using the cell culture vessel manufactured will be described. On the day before corneal epithelial cells were cultured, NIH-3T3 cells treated with mitomycin C (10 µg/ml) at 37°C for 2 hours were seeded as feeder cells on the lower layer of the cell culture vessel so as to become 2 × 10⁴/cm². On the next day after NIH-3T3 cells were seeded, corneal epithelial cells were collected from the limbus corneae of a rabbit eyeball purchased from Funakoshi Corp. by a typical method, and seeded on the upper layer of the cell culture vessel so as to become 4 × 10⁴/cm². As a culture solution, a KCM culture medium including 5% FBS typically used in the culture of epithelial cells was used. The culture solution was exchanged with a fresh culture solution on both the upper layer and the lower layer of the cell culture vessel once on days 5, 7 and 9 to 16 of the culture initiation. From day 9 on, the culture solution was exchanged with a fresh culture solution every 24 hours.

### <Method of Peeling-Off Corneal Epithelial Tissues>

On day 16 of the culture, the tissues were peeled off and recovered. The lower layer of the closed-system cell culture vessel was filled with dispase (200 U/ml) and the tissues were treated with dispase at 37°C for 1 hour, and then the tissues were peeled off.

### <Method of Measuring Number of Cells in Corneal Epithelial Tissue>

On day 16 of the culture, a 0.25% trypsin solution was used to recover cells from the cell culture vessel and the cells were stained with trypan blue, and then the number of cells was measured by a cell number counting device (TC10, manufactured by Bio-Rad Corp.) and the number of cells per culture area of the cell culture vessel was calculated.

### <Preparation of Tissue Fragment of Corneal Epithelial Tissue, Tissue Fragment Staining, Immunohistochemical Staining and Method of Forming Colony>

On day 16 of the culture, a frozen embedding was performed while the corneal epithelial cells were adhered to the material permeable film according to the typical method. A fragment of microtome having a thickness of 10 µm was prepared from the frozen embedded tissue. The prepared fragment was used to perform a hematoxylin-eosin staining, a nuclear staining and an immunohistochemical staining by typical methods. In the immunohistochemical staining, an anti-pan-CK antibody (clone name: Kspan1-8), an anti CK3 antibody (clone name: AE5), an anti-claudin (1; claudin) antibody (clone name: A10) and an anti-p63 antibody (clone name: 4A4) were used.

In Example 1 and Comparative Example 1, number of p63 positive cells/number of total cells was obtained from five fragments to calculate a p63 positive cell rate. In order to obtain a colony formation rate, a 0.05% trypsin solution was used to prepare a cell suspension liquid from the cell sheet prepared, 2000 cells in the cell suspension liquid were seeded on a 10 cm-dish, on which the NIH-3T3 cells had been previously seeded so as to become 2 × 10⁴/cm², and the cells were cultured in a KCM culture medium for about 10 days.

### (Comparative Example 1)

An experiment was performed in the same manner as in Specific Example 1, except that a commercially available cell culture insert for 6-well plates (an open-system culture vessel) was used as the cell culture vessel, the number of corneal epithelial cells seeded was 2 × 10⁴/cm², and the number of culture days was 14 days.

### (Results of Specific Example 1 and Comparative Example 1)

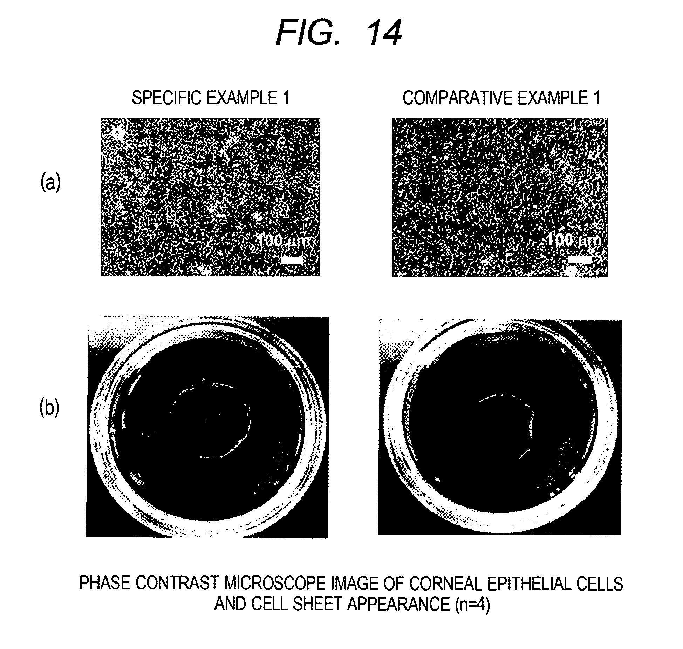

FIGS. 14 are views illustrating FIG. 14 (a) a phase contrast microscope image and FIG. 14 (b) an appearance of the cell sheet in Specific Example 1 shown at the left thereof and Comparative Example 1 shown at the right thereof. In Specific Example 1, it was possible to peel off and recover the cell sheet without any damage.

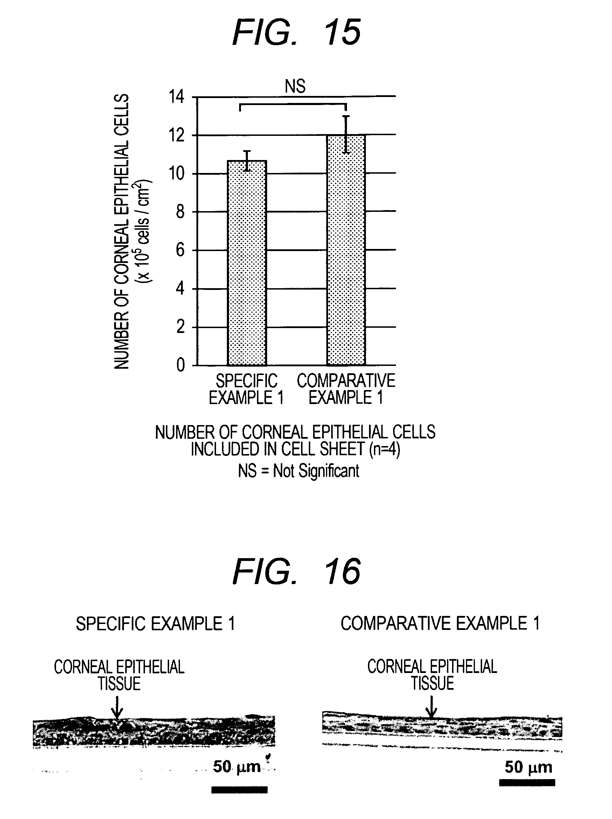

FIG. 15 is a view illustrating the number of cells included in the cell sheet. Specific Example 1 has the number of cells, which is almost equivalent to that in Comparative Example 1, and as a result of a significant difference test, there is no significant difference in both the sides.

FIG. 16 is a view illustrating a hematoxylin-eosin (HE) staining image of corneal epithelial tissue fragment in Specific Example 1 at the left thereof and Comparative Example 1 at the right thereof. In Specific Example 1, a corneal epithelial tissue in which the cell layer is multilayered to 3 layers or more may be confirmed in the same manner as in Comparative Example 1.

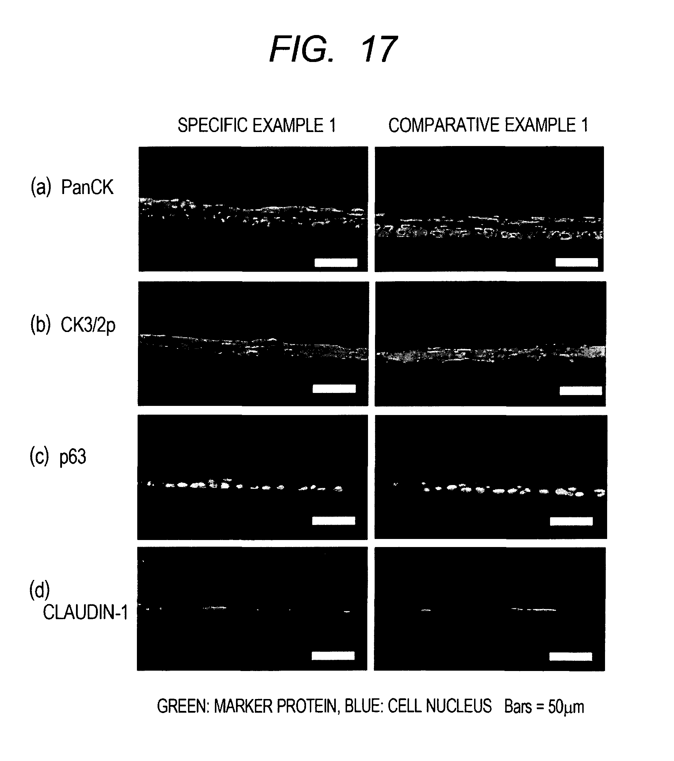

FIGS. 17 are views illustrating immunohistochemical staining images in each of Specific Example 1 illustrated in the left side thereof and Comparative Example 1 illustrated in the right side thereof in the same manner as above. In Specific Example 1, it may be confirmed that the CK protein family (PanCK) expressed in the epithelial cells illustrated in FIG. 17 (a) of the same drawing was expressed in all the cells, the CK3 expressed in the differentiated corneal epithelial cells was expressed in cells other than in the base layer as illustrated in FIG. 17 (b) of the same drawing, the p63 expressed in corneal epithelial stem cells · precursor cells was expressed in cells in the base layer as illustrated in FIG. 17 (c) of the same drawing, the claudin 1, which is a closed binding protein necessary for the barrier function of the epithelial tissue, was expressed in the outermost layer as illustrated in FIG. 17 (d) of the same drawing, and the cell sheet prepared in Specific Example 1 had a function as a corneal epithelial tissue. Further, bars illustrated in each of the photos in FIGS. 17 indicate 50 µm.

It is required that the corneal epithelial tissue prepared in the cell culture vessel in the present Example has the same quality as those prepared in the cell culture insert for 6-well plates (open-system culture vessel). From the results in FIGS. 14 to 17, it may be clearly known that Specific Example 1 had the same quality as Comparative Example 1 and a sufficiently multilayered corneal epithelial tissue had been prepared, and the results show that the cell culture vessel of the present Example shows that the vessel is appropriate for the preparation of a corneal epithelial tissue.

### (Specific Example 2)

### <Temperature Responsive Polymer Treatment for Closed-System Culture Vessel and Corneal Epithelial Cell Culture>

A temperature responsive culture surface was prepared by electron-beam polymerization of N-isopropyl acrylamide which is a monomer for temperature responsive polymers on the cell culture surface of the closed-system culture vessel. It was confirmed that the corneal epithelial cells had been normally attached to and detached from the present culture surface, and then a corneal epithelial tissue was prepared in the same manner as in Specific Example 1.

### <Peeling off Corneal Epithelial Cell Sheet>

On day 16 of the culture, the culture solution was exchanged with a fresh culture solution at room temperature, and the culture solution was allowed to stand at room temperature (about 25°C) for 30 minutes. Thereafter, as a supporting film, a hydrophilic PVDF membrane (manufactured by Millipore Corp.) cut in a doughnut shape was used to peel off and recover the cell sheet from the culture surface.

### <Preparation of Tissue Fragment of Corneal Epithelial Tissue, Tissue Fragment Staining, Immunohistochemical Staining and Method of Forming Colony>

Experiments were performed in the same manner as in Specific Example 1.

### (Comparative Example 2)

Experiments were performed in the same manner as in Comparative Example 1, except that a cell culture insert (manufactured by CellSeed Inc.) for temperature responsive polymer treating agent 6-well plates was used as the cell culture vessel.

### (Results of Specific Example 2 and Comparative Example 2)

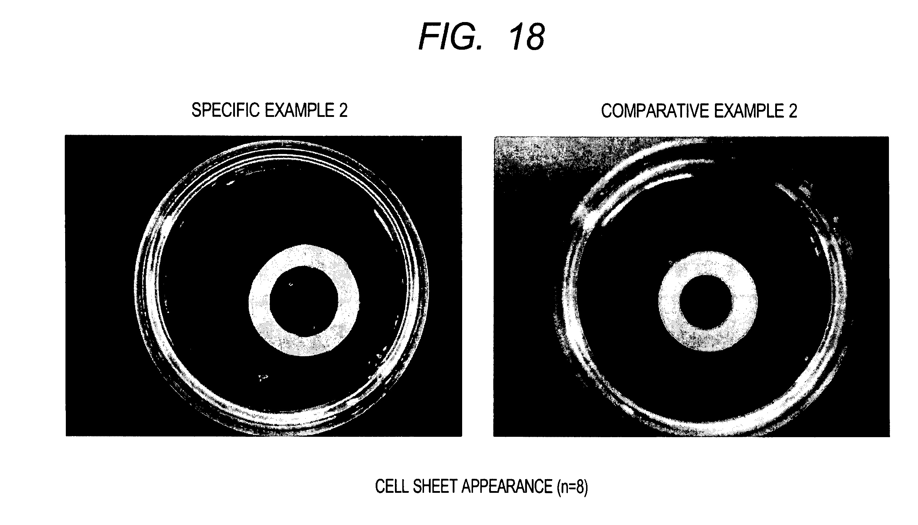

FIG. 18 is a view illustrating appearances of peeled and recovered cell sheets in Specific Example 2 at the left thereof and Comparative Example 2 at the right thereof. In Specific Example 2, it was possible to achieve peeling-off without any damage.

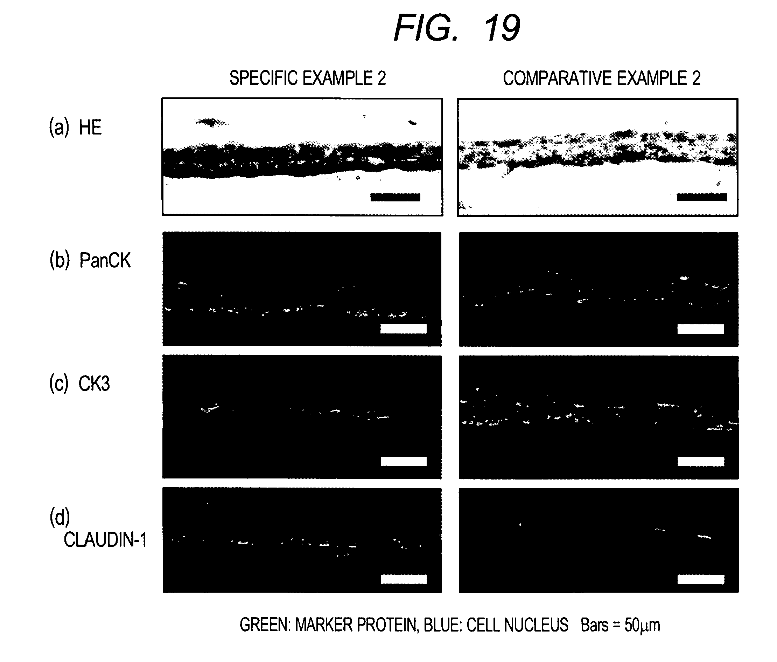

FIGS. 19 are views illustrating a hematoxylin-eosin (HE) staining image (FIG. 19 (a) of the same drawing) and an immunohistochemical staining image of each corneal epithelial tissue fragment in Specific Example 2 at the left thereof and Comparative Example 2 at the right thereof. In Specific Example 2, the CK protein family expressed in the epithelial cells was expressed in all the cells as illustrated in FIG. 19 (b) of the same drawing, the CK3 expressed in the differentiated corneal epithelial cells was expressed in cells other than in the base layer as illustrated in FIG. 19 (c) of the same drawing, and the claudin 1, which is a closed binding protein necessary for the barrier function of the epithelial tissue, was expressed in the outermost layer as illustrated in FIG. 19 (d) of the same drawing.

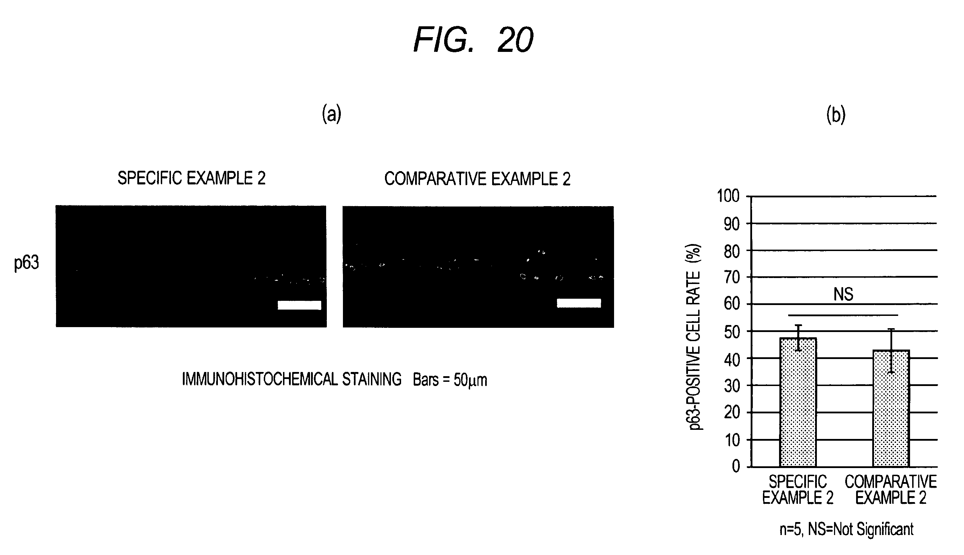

FIGS. 20 are views illustrating the presence of corneal epithelial stem cells · precursor cells on the corneal epithelial tissue fragment and the positive cell rate, in Specific Example 2 and Comparative Example 2. In Specific Example 2, the p63 expressed in corneal epithelial stem cells · precursor cells was expressed in cells in the base layer as illustrated in FIG. 20 (a) of the same drawing, and the positive cell rate was 30% or more in Specific Example 2 and Comparative Example 2 with no significant difference therebetween, as described in FIG. 20 (b) of the same drawing.

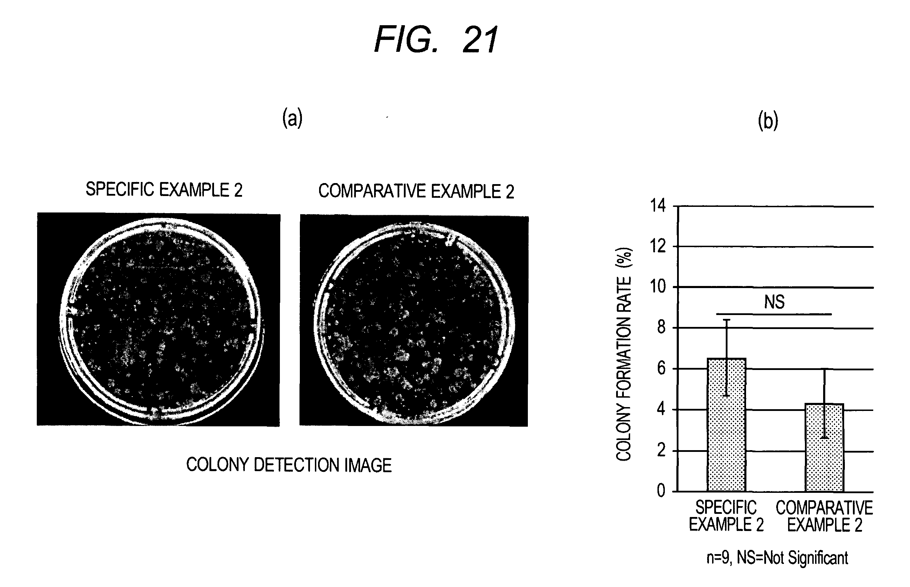

FIGS. 21 are views illustrating a colony detection image (FIG. 21 (a) of the same drawing) derived from corneal epithelial stem cells · precursor cells included in the cell sheet and a colony formation rate (FIG. 21(b) of the same drawing) in Specific Example 2 and Comparative Example 2. There was no significant difference between Specific Example 2 and Comparative Example 2, and the rate was 3% or more in both cases.

### Example 2

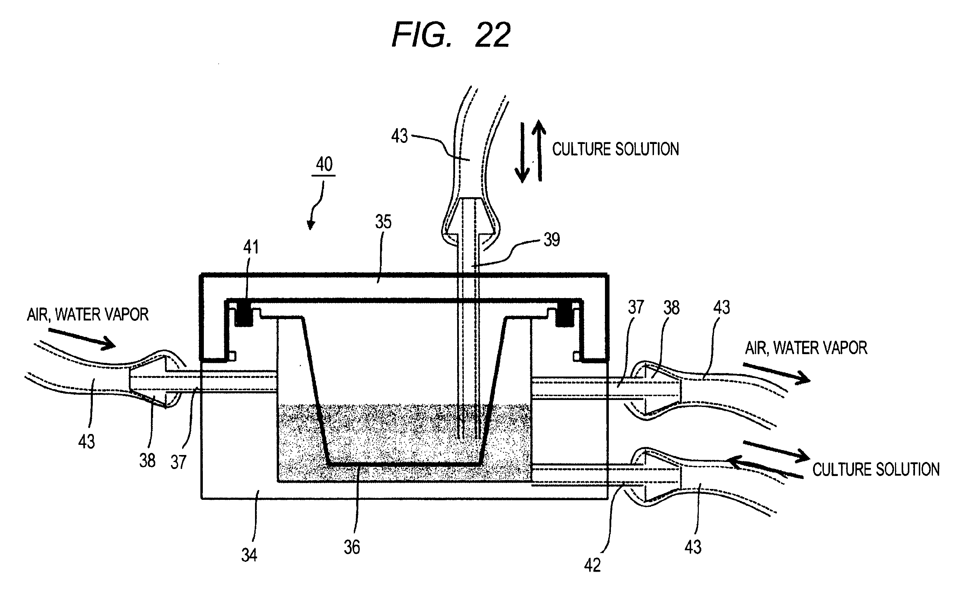

The configuration of the cell culture vessel of Example 2 will be described with reference to FIGS. 22 to 24.

### <Structure of Cell Culture Vessel>

FIG. 22 is a view illustrating an example of the structure (cross-sectional view) of a cell culture vessel in Example 2. A cell culture vessel 40 is a regular quadrilateral vessel and formed of a plastic having plasticity and rigidity, such as polycarbonate, polystyrene, polypropylene and the like. A frame body 34 and a cover 35 constituting the vessel are formed by injection molding and the like, and are designed as a structure capable of inserting an insert vessel 36 therein. As the insert vessel, a commercially available vessel may be used, and an available product, such as products manufactured by BD Corp., Corning Inc., Greiner, Inc., and the like, is used without any limitation. In the cover 35 or the frame body 34, an elastic member 41, such as an O-ring and the like, is provided, and accordingly, particles containing air or bacteria are not incorporated from the outside thereof. The connection of the cover 35 to the frame body 34 may be fixed by a screw thread provided on the cover 35 and the frame body 34, but is not limited thereto.

A duct 37 having a protruding structure 38 for injecting and discharging air and water vapor is provided in the frame body 34. A tube 43 is mounted on the tip of the duct 37. The position of the duct 37 in the frame body needs to be varied depending on the amount of the culture solution introduced into the vessel, but may be higher than the surface of the culture solution. In addition, a duct 42 having a protruding structure for injecting and discharging the culture solution through a tube 43 is provided in the frame body 34. It is preferred that the duct 42 is installed such that the bottom surface of the frame body 34 is at the same height as the lowest section of the internal diameter of the duct 42. By doing this, it is possible to efficiently discharge the culture solution. When the culture solution is all exchanged, the frame body 34 may be suitably inclined.

A duct 39 having a protruding structure for injecting and discharging the culture solution into and out of the insert vessel is provided in the cover 35. It is preferred that the duct 39 is disposed such that the observation of cells is not disturbed. The duct 39 may be long enough so as not to touch the bottom surface of the insert vessel.

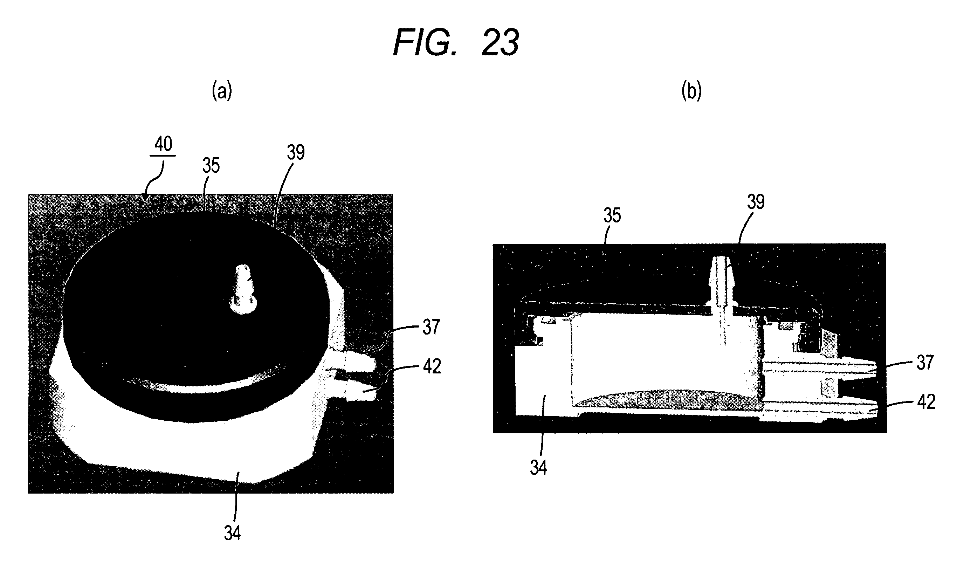

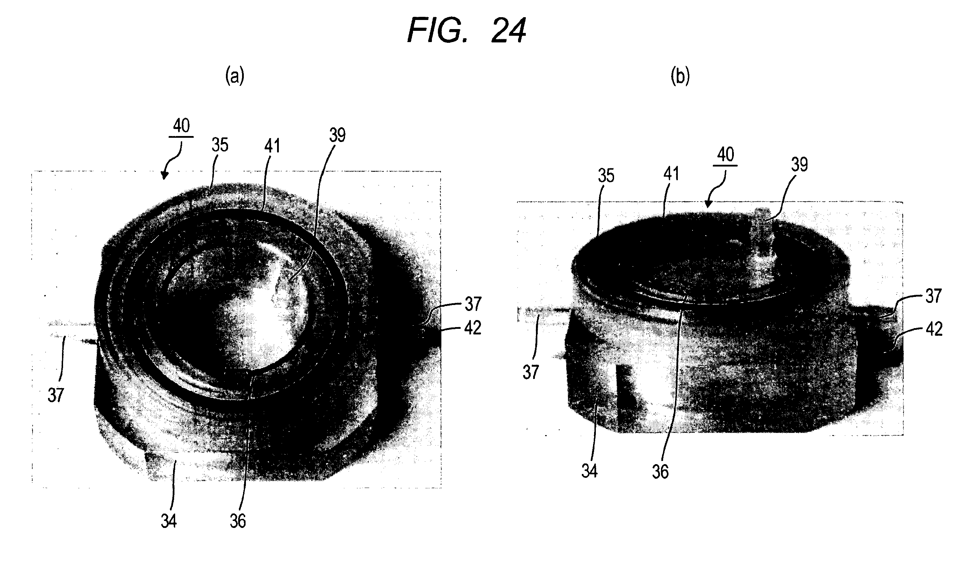

FIGS. 23 illustrate stereoscopic views of the schematic view illustrated in FIG. 22. FIG. 23 (a) of the same drawing illustrates an oblique stereoscopic structure, and FIG. 23 (b) of the same drawing illustrates a cross-sectional stereoscopic structure. FIGS. 24 illustrate are appearances of a trial product of the cell culture vessel of the present example experimentally produced. FIG. 24 (a) of the same drawing illustrates a photo viewed in the upper direction, and FIG. 24 (b) of the same drawing illustrates a photo viewed in an oblique direction. The regenerated tissues may be readily recovered by separating the cover 35 from the frame body 34.

### Example 3

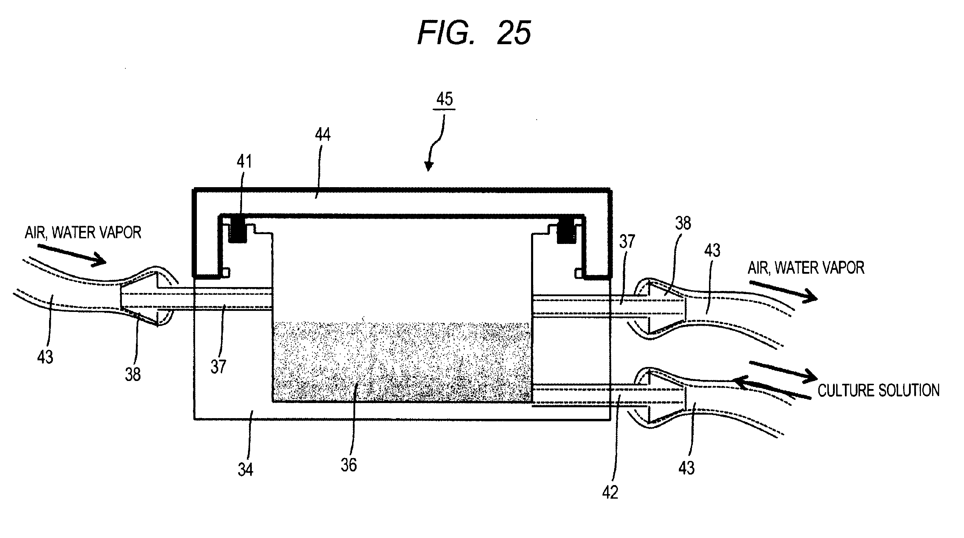

FIG. 25 illustrates the configuration of a cell culture vessel of Example 3. In the same drawing, portions having the same numerals as those in FIG. 22 indicate the same objects.

### <Structure of Cell Culture Vessel>

As illustrated in FIG. 25, in the case of a one-layer culture, the basic structure is the same as in Example 2, but the culture solution accumulated in the internal space 46 of the frame body 34 is used to culture cells by means of a cell culture vessel 45 which freshly uses a cover 44 without a duct.

Further, a regenerated tissue may be prepared in a manner that is safe and results in peace of mind by installing the cell culture vessels according to Examples 2 and 3 in the cell culture device previously described with reference to using FIG. 4.

According to the cell culture vessels and cell culture devices in various examples described above, it is possible to transfer a waste culture solution into the cell culture vessel without generating any liquid leakage by connecting a tube composed of an elastic member having an internal diameter appropriate for a protrusion directly to the protrusion, and to automatically culture cells in a completely closed-system. By culturing cells in a completely closed-system, entry of particles containing microorganisms from outside the cell culture space may be suppressed and cells may be cultured in a manner that is safe and results in peace of mind.

Further, when the culture solution is discharged from the cell culture vessel after the completion of cell culture, the culture solution may be discharged without inserting an apparatus for recovering a culture solution into the cell culture vessel, and thus entry of particles containing microorganisms from outside the cell culture space may be suppressed and cells after the culture may be recovered in a manner that is safe.

### Industrial Applicability

The present invention is useful as a cell culture vessel and a cell culture device using the same.

### Reference Signs List

| | |
|---|---|
| 1 | Cell culture vessel |
| 2 | Frame body |
| 3, 4 and 5 | Circular sections |
| 6 | Gas permeable film |
| 7 | Material permeable film |
| 8 | Gas permeable film |
| 9 | Protruding structure section |
| 10 | Tube |
| 11, 12 | Protruding structure section |
| 14 | Culture solution duct |
| 15 | Cell culture device |
| 16 | Culture stage |
| 17 | Actuating section of rotation movement |
| 18 | Liquid transferring pipe |
| 19 | Waste solution pipe |
| 20 | Control section |
| 21 | Culture solution storage section |
| 22 | Waste solution receiving section |
| 23 | Cooling box |
| 24 | XYZ movable axis |
| 25 | Cell observing apparatus |
| 26 | Member which closes the tube |
| 28 | Culture solution holding section |
| 34 | Frame body |
| 35 | Cover |
| 36 | Insert vessel |
| 37, 39 and 42 | Duct |
| 38 | Protrusion |
| 40 and 45 | Cell culture vessel |
| 41 | Elastic member |
| 43 | Tube |
| 44 | Cover |

## Claims

1. A cell culture vessel mounted to a cell culture device to hold and culture cells, the vessel comprising:
a culture solution holding section that holds a culture solution;
a protruding structure section that is for supplying and discharging the culture solution; and
a culture solution duct that passes from the protruding structure section to the culture solution holding section.

2. The cell culture vessel according to claim 1, wherein a pair of the protruding structure sections and a pair of the culture solution ducts are provided.

3. The cell culture vessel according to claim 1, wherein at least one surface of the culture solution holding section has a gas permeable film in order to hold and culture cells.

4. The cell culture vessel according to claim 1, wherein the protruding structure section is capable of being connected to a tube formed of an elastic member.

5. The cell culture vessel according to claim 1, wherein the culture solution holding section has a double-layered structure in which a material permeable film is interposed, and the protruding structure section and the culture solution duct are formed on each layer of the double-layered structure.

6. The cell culture vessel according to claim 1, wherein both surfaces of the culture solution holding section have a gas permeable film in order to hold and culture cells.

7. The cell culture vessel according to claim 3, wherein the other surface of the culture solution holding section is formed integrally with the frame body of the cell culture vessel.

8. The cell culture vessel according to claim 3, wherein the gas permeable film is welded to at least one surface of the culture solution holding section to include a protruding section protruding to a part of the outer perimeter thereof.

9. The cell culture vessel according to claim 3, wherein the protruding structure section protrudes in a direction vertical to a film surface of the gas permeable film from an upper surface of the frame body of the cell culture vessel.

10. The cell culture vessel according to claim 3, wherein the protruding structure protrudes in a direction parallel to a film surface of the gas permeable film from a side surface of the frame body of the cell culture vessel.

11. The cell culture vessel according to claim 2, wherein the culture solution holding section has an approximately circular shape, and a pair of the culture solution ducts are connected to an opposing position having a circular shape.

12. The cell culture vessel according to claim 1, wherein the culture solution holding section comprises a frame body that holds the culture solution and a cover that covers an upper section of the frame body, and the culture solution duct is provided in the frame body.

13. The cell culture vessel according to claim 2, wherein the culture solution holding section comprises a frame body that holds the culture solution and a cover that covers an upper section of the frame body, and one of a pair of the culture solution ducts is provided in the frame body and the other is provided in the cover.

14. A cell culture device that cultures cells in a cell culture vessel, the device comprising:
a culture stage that holds the cell culture vessel;
a culture solution storage section for storing a culture solution;
a waste solution receiving section for collecting a waste solution;
a liquid transferring pipe for supplying the culture solution from the culture solution storage section to the cell culture vessel; and
a waste solution pipe that recovers a waste solution from the cell culture vessel and transfers the waste solution to the waste solution receiving section,
wherein the cell culture vessel comprises a culture solution holding section that holds the culture solution, a protruding structure section that is for supplying and discharging the culture solution, and a culture solution duct that passes from the protruding structure section to the culture solution holding section, and the protruding structure section is connected to one end of each of the liquid transferring pipe and the waste solution pipe.

15. The cell culture device according to claim 14, wherein a pair of the protruding structure sections and a pair of the culture solution ducts are provided.

16. The cell culture device according to claim 14, wherein the cell culture vessel has a gas permeable film on at least one surface of the culture solution holding section in order to hold and culture the cells.

17. The cell culture device according to claim 14, wherein the liquid transferring pipe and the waste solution pipe are composed of a tube formed of an elastic member.

18. The cell culture device according to claim 14, further comprising:
an actuating section that rotates so as to have a state in which the cell culture vessel mounted is horizontally maintained and a state in which the cell culture vessel mounted is vertically maintained, by rotating the culture stage.

19. The cell culture device according to claim 18, wherein the culture passage of the cell culture vessel is connected to the uppermost section and the bottom most section of the culture solution holding section while being held vertically by the actuating section.

20. The cell culture device according to claim 15, wherein the culture solution holding section comprises a frame body that holds the culture solution and a cover that covers an upper section of the frame body, and one of a pair of the culture solution ducts is provided in the frame body and the other is provided in the cover.
